# NOUVEAU FASCICULE DE BREVET EUROPEEN

(11) **EP 1 132 079 B2**
(45) Date de publication et mention de la décision concernant l'opposition: **05.05.2010**
(45) Mention de la délivrance du brevet: 16.03.2005
(21) Numéro de dépôt: 01400095.4
(22) Date de dépôt: 15.01.2001
(51) Int. Cl.: A61K 8/49, A61K 8/44, A61K 8/73, A61Q 5/02

(54) **Composition de lavage des matières kératiniques à base d'ester de sorbitan faiblement éthoxylé**
Waschmittel für keratinische Materialien die ein niedrig ethoxylierten Ester des Sorbits enthalten
Washing composition for keratinic materials comprising a low-ethoxylated sorbitan ester

(30) Priorité: 21.01.2000 FR 0000790
(43) Date de publication de la demande: 12.09.2001
(73) Titulaire: L'Oréal, 75008 Paris (FR)
(72) Inventeur: Maubru, Mireille, 78400 Chatou (FR); Decoster, Sandrine, 95210 Saint-Gratien (FR); Beauquey, Bernard, 92110 Clichy (FR)
(74) Mandataire: Casalonga, Axel

(56) Documents cités:
- EP-A- 0 642 782
- EP-A2- 0 160 269
- EP-A2- 7 280 475
- WO-A1-95/03782
- DE-A1- 3 839 349
- FR-A- 2 753 373
- US- - 3 819 524
- US-A- 3 819 524
- US-A- 3 988 255
- US-A- 4 772 427
- US-A- 5 482 705
- US-A- 5 911 981

## Description

La présente invention est relative à des compositions de lavage des matières kératiniques, et en particulier des cheveux et/ou de la peau, à base d'ester de sorbitan faiblement éthoxylé et au procédé de lavage mettant en oeuvre ces compositions.

Il est connu d'utiliser des tensioactifs non ioniques, tels que les esters de sorbitan fortement polyoxyéthylénés dans des shampooings. Ces esters de sorbitan font office de détergents auxiliaires dans ce type de formulation.

Le brevet belge No 840667 (Johnson & Johnson) décrit des compositions détergentes et shampooings à base d'associations de tensioactifs non ioniques comme les esters de sorbitan fortement éthoxylés peu irritants pour les yeux et ayant un pouvoir moussant satisfaisant. Ces esters de sorbitan comportent à peu près une quarantaine d'unités d'oxyde d'éthylène, le composé spécialement préféré comportant 44 unités d'oxyde d'éthylène.

Dans ce brevet, les compositions détergentes contiennent obligatoirement au moins un dérivé polyoxyéthylénique d'une base hydrophobe comme agent tensioactif non ionique, un agent tensioactif anionique et une bétaïne tensio-active. C'est l'association de ces trois composés qui confèrent à la composition une mousse d'une bonne stabilité peu irritante pour les yeux.

De même, la demande de brevet EP 0815851A2 décrit des compositions, notamment des shampooings comportant des esters de sorbitan fortement oxyéthylénés, avec au moins 20 unités d'oxyde d'éthylène, en association avec des tensioactifs anioniques afin de réduire l'irritation oculaire en présence de tensioactifs anioniques.

La demande de brevet EP 0453238 A1 décrit des compositions cosmétiques utilisées sous forme de shampooings conférant de la douceur et une bonne capacité de moussage. Ces compositions comprennent des agents tensioactifs de différents types et notamment des esters de sorbitan comme le Polysorbate 20 et le Polysorbate 80 comportant approximativement 20 moles d'oxyde d'éthylène. Ces compositions comprennent au moins un composé tensioactif non ionique en association avec au moins un tensioactif anionique et un tensioactif amphotère.

Toutes ces compositions de l'art antérieur présentent la caractéristique d'être difficiles à épaissir, ce qui conduit à des qualités d'usage médiocres: mauvaise répartition du produit, émulsification difficile, toucher chargé.

Des compositions comprenant des esters de sorbitan et des agents tensioactifs anioniques dans différents rapports pondéraux agent tensioactif anionique/ester de sorbitan sont également décrites dans les documents US 3 988 255, US 3 819 524, US 4 772 427, FR 2 753 373 et US 5 482 705.

La demanderesse a découvert, de façon surprenante, qu'il était possible de formuler des compositions de lavage des matières kératiniques, notamment des shampooings présentant une bonne tolérance oculaire et produisant un épaississement amélioré des formules de shampooings en utilisant un ester de sorbitan faiblement oxyéthyléné ayant un nombre de moles d'oxyde d'éthylène inférieur ou égal à 10. Ces résultats sont obtenus en particulier pour un rapport pondéral agent tensioactif détergent anionique/ester de sorbitan compris entre 0,5 et 5.

L'invention a pour objet des compositions de lavage des matières kératiniques telles que définies dans les revendications 1 et 2.

L'invention a également pour objet l'utilisation d'au moins un ester de sorbitan ayant un nombre de moles d'oxyde d'éthylène inférieur ou égal à 10 dans le but de réduire le potentiel irritant pour les yeux et d'améliorer l'épaississement des compositions de lavage des matières kératiniques contenant un tensioactif détergent.

Un autre objet de l'invention est un procédé de lavage des matières kératiniques.

D'autres objets de l'invention apparaîtront à la lecture de la description et des exemples qui suivent.

Les compositions de lavage des matières kératiniques conformes à l'invention comprennent, dans un milieu cosmétiquement acceptable contenant au moins un agent tensioactif détergent anionique, au moins un ester de sorbitan d'acide gras en C₈-C₃₀, de préférence en C₈-C₂₀, saturé ou insaturé, linéaire ou ramifié, en un rapport en poids agent tensioactif détergent anionique/ester de sorbitan d'acide gras en C₈-C₃₀ compris entre 0,5 et 5, et au moins un polymère cationique ou un tensioactif amphoter, la quantité d'agent tensioactif anionique étant comprise entre 5 et 40% en poids par rapport au poids total de la composition.

On utilise de préférence des mono-esters de sorbitan oxyéthyléné avec un nombre de moles d'oxyde d'éthylène égal ou inférieur à 10.

Le nombre de moles d'oxyde d'éthylène est compris de préférence entre 3 et 8 moles d'oxyde d'éthylène.

Les esters de sorbitan préférés sont le mono-laurate de sorbitan oxyéthyléné (40E) ou polysorbate 21, le mono-stéarate de sorbitan oxyéthyléné (40E) ou polysorbate 61, le mono-oléate de sorbitan oxyéthyléné (50E) ou polysorbate 81.

Selon la présente invention, l'ester de sorbitan peut être présent dans la composition de lavage des matières kératiniques dans des proportions de 0,5 à 20 %, et préférentiellement de 2 à 15 % en poids par rapport au poids total de ladite composition.

La titulaire a découvert que l'adjonction d'ester de sorbitan faiblement oxyéthyléné, susdéfini, permettait de réduire de façon surprenante le caractère irritant, notamment au niveau oculaire, des compositions de lavage contenant des tensioactifs détergents tels que les tensioactifs anioniques, en particulier les alkylsulfates et les alkyléthersulfates, susceptibles de provoquer des irritations lorsqu'ils sont utilisés seuls.

Parmi les agents tensioactifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates et alkyléther phosphates; les acylsarcosinates, les acyliséthionates et les N-acyltaurates.

Parmi les agents tensioactifs anioniques, on peut également citer les sels d'acides gras tels que les sels des acides oléique, ricinoléique, palmitique, stéarique; les acides d'huile de coprah ou d'huile de coprah hydrogénée; les acyl lactylates. Les radicaux acyle ou alkyle comportent généralement de 8 à 30 atomes de carbone.

On peut également utiliser des agents tensioactifs, tels que les acides alkyl ou alkylaryl éther carboxyliques polyoxyalkylénés ou leurs sels, les acides alkylamido éther carboxyliques polyoxyalkylénés ou leurs sels, les acides d'alkyl D-galactoside uroniques ou leurs sels.

Le tensioactif anionique est utilisé dans des proportions comprises entre 5 et 40 % en poids, par rapport au poids total de la composition.

La composition selon l'invention ne contient pas de préférence de savons.

La composition de la présente invention peut contenir en outre un ou plusieurs détergents choisis parmi les tensioactifs non-ioniques différents des esters de sorbitan faiblement oxyéthylénés sus-définis, dans des proportions suffisantes pour conférer à la composition des propriétés détergentes.

Les agents tensioactifs non-ioniques additionnels sont plus particulièrement choisis parmi les alcools ou les alkylphénols ou les acides gras polyéthoxylés, polypropoxylés ou polyglycérolés, à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30, à l'exception des esters de sorbitan d'acide gras en C₈-C₃₀ oxyéthyléné avec un nombre de moles d'oxyde d'éthylène égal ou inférieur à 10.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 12 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensioactifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀) amidoalkyl( C₁-C₆)bétaïnes ou les alkyl(C₈-C₂₀)amidoalkyl(C₁-C₆) sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.

Les tensioactifs détergents sont présents généralement dans des proportions comprises entre 5 et 40% en poids par rapport au poids total de la composition.

Les compositions peuvent également contenir des agents tensioactifs cationiques utilisés de préférence dans des proportions comprises entre 0,001 à 5% en poids, par rapport au poids total de la composition.

Les agents tensioactifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par exemple chlorures) de tétraalkylammonium comme, par exemple, les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyl diméthyl (myristyl acétate) ammonium, commercialisés sous la dénomination de "CEPHARYL 70" par la Société VAN DYK.

On peut également utiliser les sels (chlorures ou méthylsulfate, notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium, et leurs mélanges.

Les radicaux acyle proviennent plus particulièrement d'une huile végétale comme l'huile de palme ou de tournesol.

La concentration des tensioactifs additionnels autres que les tensioactifs anioniques, peut varier de 0 à 30 % et de préférence de 0,5 à 15% en poids, par rapport au poids total de la composition.

Les compositions selon l'invention peuvent contenir également des silicones, à savoir des huiles de polyorganosiloxanes, ou des gommes ou des résines de polyorganosiloxanes, telles que sous la forme de solutions dans des solvants organiques ou encore sous forme d'émulsions ou de microémulsions.

Parmi les polyorganosiloxanes pouvant être utilisés conformément à la présente invention, on peut citer à titre non limitatif :
- les silicones volatiles: celles-ci possèdent un point d'ébullition compris entre 60°C et 260°C. Elles sont choisies parmi les silicones cycliques contenant de 3 à 7 atomes de silicium et de préférence 4 à 5.
- les silicones non volatiles : elles sont constituées principalement par les polyalkylsiloxanes, les polyarylsiloxanes, les polyalkylarylsiloxanes, les gommes de silicone, les résines de silicone ou leurs mélanges et les silicones organomodifiées.

On utilise plus particulièrement les polydiméthylsiloxanes, les silicones aminées et les silicones oxyalkylénées.

Les polyorganosiloxanes peuvent être utilisés dans les compositions de l'invention dans des proportions comprises entre 0,01 et 20% en poids et de préférence entre 0,1 et 10 % en poids par rapport au poids total de la composition.

Les compositions de l'invention peuvent contenir en outre un polymère amphotère.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celles-ci, des groupements ammonium quaternaire. Leur masse moléculaire peut varier par exemple de 1 500 à 10 000, et en particulier de 2 000 à 5 000 environ.

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone/acrylate ou méthacrylate de dialkylaminoalkyle quaternisés ou non, tels que les polymères décrits en détail dans les brevets français 2 077 143 et 2 393 573.
(2) Les dérivés d'éthers de cellulose comportant des groupements ammonium quaternaire décrits dans le brevet français 1 492 597.
(3) Les dérivés de cellulose cationiques, tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées, notamment avec un sel de méthacryloyléthyl triméthyl ammonium, de méthacrylamidopropyl triméthyl ammonium ou de diméthyldiallylammonium.
(4) Les polysaccharides et notamment gommes de guar cationiques décrits plus particulièrement dans les brevets US 3 589 578 et 4 031 307.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer, par exemple, les polymères acide adipique/diacoylamino-hydroxyalcoyldialcoylène-triamine dans lesquels le radical alcoyle comporte de 1 à 4 atomes de carbone et désigne de préférence méthyle, éthyle, propyle. De tels polymères sont notamment décrits dans le brevet français 1.583.363.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères de méthyl diallyl amine ou de diméthyl diallyl ammonium, notamment ceux décrits dans le brevet français 2.080.759 et dans son certificat d'addition 2.190.406.
(10) Les polymères de diammonium quaternaire décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) Les polymères de polyammonium quaternaire notamment décrits dans la demande de brevet EP-A-122 324.
(12) Les homopolymères ou copolymères dérivés des acides acrylique ou méthacrylique et comportant des motifs

   CH₂-CH Rₐ-CO-O- A₁- NRₑR_{f},

   CH₂-CHRₐ-CO-O-A₁-N⁺R_{b}R_{c}R_{d}-X⁻

   et/ou

   CH₂-CHRₐ-CO-NH-A₁-N⁺R_{b}R_{c}R_{d}, X⁻

   dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
(13) Les polymères quaternaires de vinylpyrrolidone et de vinylimidazole, tels que, par exemple, les produits commercialisés sous les dénominations "LUVIQUAT FC 905", "LUVIQUAT FC 550" et "LUVIQUAT FC 370" par la société BASF.
(14) Les polyamines comme le "POLYQUART H" vendu par HENKEL, référencé sous le nom de « POLYETHYLENEGLYCOL TALLOW POLYAMINE » dans le dictionnaire CTFA.
(15) Les polymères réticulés de sel de méthacryloyloxyéthyl triméthylammonium, tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléhnique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère les dérivés d'éthers de cellulose comportant des groupements ammonium quaternaire, les polysaccharides et, notamment les gommes de guar cationiques et les cyclopolymères de méthyl diallyl ammonium ou de diméthyl diallyl ammonium.

Les polymères cationiques sont utilisés dans les compositions de l'invention dans des proportions comprises entre 0,001 et 20 % en poids et de préférence entre 0,05 et 5 % en poids par rapport au poids total de la composition.

Les compositions, selon l'invention, présentent un pH généralement compris entre 3 et 12, et plus particulièrement entre 4 et 8.

Le milieu cosmétiquement acceptable des compositions est constitué soit par de l'eau, soit par un ou plusieurs solvants, soit par un mélange d'eau et d'au moins un solvant choisi parmi les alcools inférieurs, les alkylèneglycols et les éthers de polyols.

Les compositions selon l'invention peuvent contenir également des agents régulateurs de viscosité, tels que des électrolytes comme le chlorure de sodium, des épaississants comme les dérivés de la cellulose, tels que par exemple la carboxyméthylcellulose, l'hydroxypropylcellulose, l'hydroxyéthylcellulose, la gomme de guar, les gommes de guar hydroxypropylées, les scléroglucanes, la gomme de xanthane, les polymères amphiphiles comportant au moins une chaîne grasse.

Les compositions selon l'invention peuvent également contenir différents adjuvants habituellement utilisés en cosmétique, tels que des parfums, des conservateurs, des synergistes de mousse, des séquestrants, des stabilisateurs de mousse, des agents propulseurs, des colorants, des agents anti-pelliculaires, des céramides, des vitamines ou provitamines, des hydroxy acides, des agents acidifiants ou alcalinisants, des huiles végétales minérales, animales, des huiles de synthèse telles que les polyisobutènes et les polydécènes, des esters d'acides gras, des pseudocéramides, des agents nacrants ou d'autres adjuvants selon l'usage envisagé.

L'invention a aussi pour objet l'utilisation dans un shampooing d'au moins un ester de sorbitan d'acide gras en C₈-C₃₀ sus-défini.

Un autre objet de la présente invention est un procédé de lavage des matières kératiniques, qui consiste dans l'application sur ces matières d'au moins une composition de la présente invention, suivie d'un rinçage des matières traitées après un éventuel temps de pose.

Les exemples qui suivent sont destinés à illustrer l'invention sans pour autant présenter un caractère limitatif.

### Exemple 1

On prépare la composition suivante:

| | |
|---|---|
| Lauryléther sulfate de sodium (C12/C14 à 70/30) à 2,2 moles d'oxyde d'éthylène | 7,0 g M.A. |
| Cocoylamidopropylbétaïne en solution aqueuse à 38 % de M.A. | 3,8 g M.A. |
| Monostéarate de sorbitan oxyéthyléné à 4 moles d'oxyde d'éthylène, commercialisé sous la dénomination "TWEEN 61" par UNIQEMA | 6,0 g |
| Alcool laurique oxyéthyléné à 2,5 moles d'oxyde d'éthylène | 1,0 g |
| Hydroxyéthylcellulose réticulée par l'épichlorhydrine et quaternisée par la triméthylamine, vendue sous la dénomination "JR 400" par la Société UNION CARBIDE | 0,2 g |
| Conservateurs | q.s. |
| Eau déminéralisée q.s.p. | 100,0 g |
| | pH 7,0 |

### Exemple 2

On prépare la composition suivante :

| | |
|---|---|
| Lauryléther sulfate de sodium (C12/C14 à 70/30) à 2,2 moles d'oxyde d'éthylène | 8,4 g M.A. |
| Monosulfosuccinate d'alcool laurique à 3 moles d'oxyde d'éthylène, sel disodique, en solution aqueuse protégée à 30 % de M.A. | 2,6 g M.A. |
| Mono-oléate de sorbitan oxyéthyléné à 5 moles d'oxyde d'éthylène, commercialisé sous la dénomination "TWEEN 81" par UNIQEMA | 5,0 g |
| Dioléate de polyéthylène glycol (55 moles d'oxyde d'éthylène) et de propylène glycol, en solution hydroglycolique, commercialisé sous la dénomination "ANTIL 141 LIQUID" par la Société GOLDSCHMIDT | 5,0 g |
| Chlorure d'hydroxypropyl guar triméthyl ammonium, vendu sous la dénomination de "JAGUAR C162" par la Société RHODIA | 0,05 g |
| Soude q.s.p. | pH 7,0 |
| Conservateurs | q.s. |
| Eau déminéralisée q.s.p. | 100,0 g |

### Exemple 3

On prépare la composition suivante:

| | |
|---|---|
| Lauryléthersulfate de sodium (C12/C14 à 70/30) à 2,2 moles d'oxyde d'éthylène | 11,8 g M.A. |
| Cocoylamidopropylbétaïne en solution aqueuse à 38 % de M.A. | 1,8 g M.A. |
| Mélange 1-(hexadécyloxy)-2-octadécanol/alcool cétylique | 2,5 g |
| Cétostéarylsulfate de sodium | 0,8 g |
| Mono-laurate de sorbitan oxyéthyléné à 4 moles d'oxyde d'éthylène, commercialisé sous la dénomination "TWEEN 21" par la Société UNIQEMA | 8,0 g |
| Polydiméthylsiloxane vendu sous la dénomination MIRASIL DM 500 000 par RHODIA CHIMIE | 1,5 g |
| Hydroxyéthylcellulose réticulée par l'épichlorhydrine et quaternisée par la triméthylamine, vendue sous la dénomination "JR 400" par la Société UNION CARBIDE | 0,4 g |
| Acide polyacrylique réticulé commercialisé sous la dénomination Carbopol 980 par GOODRICH | 0,2 g |
| Hydroxyde de sodium q.s.p. | pH 6,5 |
| Conservateurs, parfum | q.s. |
| Eau déminéralisée q.s.p. | 100,0 g |

### Exemples comparatifs

On a réalisé les compositions suivantes :
Exemple 4A (selon l'invention) : Composition contenant le tensioactif non ionique ester de sorbitan faiblement oxyéthyléné comme le TWEEN 21 (4 moles d'oxyde d'éthylène).
Exemple 4B: Composition contenant le tensioactif non ionique ester de sorbitan fortement oxyéthyléné tel que le TWEEN 20 (20 moles d'oxyde d'éthylène).

L'épaississement des formules de shampooing est nettement plus aisé lorsqu'on remplace un ester de sorbitan fortement oxyéthyléné tel que le TWEEN 20 (20 moles d'oxyde d'éthylène), classiquement utilisé dans des shampooings pour enfants, par un composé moins oxyéthyléné comme le TWEEN 21 (4 moles d'oxyde d'éthylène) et le shampooing obtenu possède par ailleurs un excellent niveau de tolérance oculaire.

### Exemple 4A (selon l'invention)

| | |
|---|---|
| Lauryléther sulfate de sodium (C12/C14 à 70/30) à 2,2 d'oxyde d'éthylène | 7,0 g M.A. |
| Cocoyl amphohydroxypropyl sulfonate de sodium en solution aqueuse à 32 %, commercialisé sous la dénomination "MIRANOL CSE" par la Société RHODIA | 12,0 g |
| Mono-laurate de sorbitan oxyéthyléné à 4 moles d'oxyde d'éthylène, commercialisé sous la dénomination "TWEEN 21" par la Société UNIQEMA | 8,0 g |
| Mono-laurate de sorbitan oxyéthyléné à 20 moles d'oxyde d'éthylène, commercialisé sous la dénomination "TWEEN 20" par la Société UNIQEMA | |
| Hydroxyéthylcellulose réticulée par l'épichlorhydrine et quaternisée par la triméthylamine, vendue sous la dénomination "JR 400" par la Société UNION CARBIDE | 0,25 g |
| Acide citrique q.s.p | pH 7,0 |
| Conservateurs | q.s. |
| Eau déminéralisée q.s.p | 100,0 g |

### Exemple 4B

| | |
|---|---|
| Lauryléther sulfate de sodium (C12/C14 à 70/30) à 2,2 moles d'oxyde d'éthylène | 7,0 g M.A. |
| Cocoyl amphohydroxypropyl sulfonate de sodium en solution aqueuse à 32 %, commercialisé sous la dénomination "MIRANOL CSE" par la Société RHODIA Mono-laurate de sorbitan oxyéthyléné à 4 moles d'oxyde d'éthylène, commercialisé sous la dénomination "TWEEN 21" par la Société UNIQEMA | 12,0 g |
| Mono-laurate de sorbitan oxyéthyléné à 20 moles d'oxyde d'éthylène, commercialisé sous la dénomination "TWEEN 20" par la Société UNIQEMA | 8,0 g |
| Hydroxyéthylcellulose réticulée par l'épichlorhydrine et quaternisée par la triméthylamine, vendue sous la dénomination "JR 400" par la Société UNION CARBIDE | 0,25 g |
| Acide citrique q.s.p. | pH 7,0 |
| Conservateurs | q.s. |
| Eau déminéralisée q.s.p | 100,0 g |

Des mesures de viscosité ont été effectuées à l'aide du viscosimètre HAAKE VT550, équipé du mobile cylindrique MV B Din, à 25 °C.

Le graphe de la figure annexée montre que la viscosité de la formule selon l'invention (exemple 4A), qui contient le "TWEEN 21", est nettement supérieure à la viscosité de la formule contenant du "TWEEN 20", qui est totalement liquide (l'exemple 4B).

## Revendications

1. Composition de lavage des matières kératiniques **caractérisée par le fait qu'**elle comprend, dans un milieu aqueux cosmétiquement acceptable contenant au moins un agent tensioactif détergent anionique, au moins un ester de sorbitan d'acide gras en C₈-C₃₀, saturé ou insaturé, linéaire ou ramifié, oxyéthyléné avec un nombre de moles d'oxyde d'éthylène inférieur ou égal à 10, en un rapport en poids agent tensioactif détergent anionique/ester de sorbitan compris entre 0,5 et 5, la quantité d'agent tensioactif détergent anionique étant comprise entre 5 et 40% en poids par rapport au poids total de la composition, et au moins un polymère cationique.

2. Composition de lavage des matières kératiniques **caractérisée par le fait qu'**elle comprend, dans un milieu aqueux cosmétiquement acceptable contenant au moins un agent tensioactif détergent anionique, au moins un ester de sorbitan d'acide gras en C₈-C₃₀, saturé ou insaturé, linéaire ou ramifié, oxyéthyléné avec un nombre de moles d'oxyde d'éthylène inférieur ou égal à 10, en un rapport en poids agent tensioactif détergent anionique/ester de sorbitan compris entre 0,5 et 5, la quantité d'agent tensioactif détergent anionique étant comprise entre 5 et 40% en poids par rapport au poids total de la composition, et au moins un tensioactif amphotère.

3. Composition selon la revendication 1 ou 2, **caractérisée par le fait qu'**elle contient au moins un ester de sorbitan d'acide gras en C₈-C₂₀ oxyéthyléné avec un nombre de moles d'oxyde d'éthylène inférieur ou égal à 10.

4. Composition selon la revendication 1, ou 2 ou 3, **caractérisée par le fait que** l'ester de sorbitan oxyéthyléné contient 3 à 8 moles d'oxyde d'éthylène.

5. Composition selon l'une quelconque des revendications 1 à 4, **caractérisée par le fait que** l'ester de sorbitan est un mono-ester de sorbitan.

6. Composition selon l'une quelconque des revendications 1 à 5, **caractérisée par le fait que** l'ester de sorbitan est choisi parmi le mono-laurate de sorbitan oxyéthyléné (40E), le mono-stéarate de sorbitan oxyéthyléné (40E) et le mono-oléate de sorbitan oxyéthyléné (5OE).

7. Composition selon l'une quelconque des revendications 1 à 6, **caractérisée par le fait que** l'ester de sorbitan est présent dans des proportions de 0,5 à 20%, et préférentiellement de 2 à 15% en poids de la ladite composition.

8. Composition selon l'une quelconque des revendications 1 à 7, **caractérisée par le fait qu'**elle comprend en outre un agent tensioactif détergent choisi parmi les tensioactifs non-ioniques différents des esters de sorbitan d'acide gras en C₈-C₃₀ oxyéthylénés avec un nombre de moles d'oxyde d'éthylène égal ou inférieur à 10, les tensioactifs amphotères ou zwitterioniques.

9. Composition selon l'une quelconque des revendications 1 à 8, **caractérisée par le fait qu'**elle contient en outre un ou plusieurs agents tensioactifs cationiques.

10. Composition selon l'une quelconque des revendications 1 à 9, **caractérisée par le fait qu'**elle contient en outre une ou plusieurs silicones.

11. Composition selon l'une quelconque des revendications 1 à 10, **caractérisée par le fait qu'**elle contient en outre un ou plusieurs polymères cationiques ou amphotères.

12. Composition selon la revendication 11, **caractérisée par le fait qu'**elle contient au moins un polymère cationique choisi parmi les dérivés d'éthers de cellulose comportant des groupements ammonium quaternaire, les polysaccharides, notamment les gommes de guar cationiques et les cyclopolymères de méthyl diallyl ammonium ou de diméthyl diallyl ammonium.

13. Composition selon l'une quelconque des revendications 1 à 12, **caractérisée par le fait qu'**elle contient en outre des agents régulateurs de viscosité, tels que des électrolytes comme le chlorure de sodium, des épaississants comme les dérivés de la cellulose, la gomme de guar, les gommes de guar hydroxypropylées, les scléroglucanes, la gomme de xanthane et les polymères amphiphiles comportant au moins une chaîne grasse.

14. Composition selon l'une quelconque des revendications 1 à 13, **caractérisée par le fait qu'**elle contient en outre un ou plusieurs adjuvants choisis parmi les parfums, les conservateurs, les synergistes de mousse, les séquestrants, les stabilisateurs de mousse, les agents propulseurs, les colorants, les agents anti-pelliculaires, les céramides, les vitamines ou provitamines, les hydroxy-acides, les agents acidifiants ou alcalinisants, les huiles végétales minérales, animales, les huiles de synthèse telles que les polyisobutènes et les polydécènes, les esters d'acides gras, les pseudocéramides et les agents nacrants.

15. Utilisation d'au moins un ester d'acide gras en C₈-C₃₀ de sorbitan ayant un nombre de moles d'oxyde d'éthylène inférieur ou égal à 10 dans le but de réduire le potentiel irritant pour les yeux et d'améliorer l'épaississement des compositions de lavage des matières kératiniques comprenant un tensioactif détergent.

16. Utilisation selon la revendication 15, dans un shampooing.

17. Procédé de lavage des matières kératiniques, **caractérisé par** l'application sur ces matières d'au moins une composition telle que définie dans l'une quelconque des revendications 1 à 14, suivie d'un rinçage des matières traitées après un éventuel temps de pose.

## Claims

1. Composition for washing keratin materials, **characterized in that** it comprises, in a cosmetically acceptable aqueous medium containing at least one anionic detergent surfactant, at least one sorbitan ester of a saturated or unsaturated, linear or branched C₈-C₃₀ fatty acid, oxyethylenated with a number of moles of ethylene oxide of less than or equal to 10, in a sorbitan ester/anionic detergent surfactant weight ratio of between 0.5 and 5, the quantity of anionic detergent surfactant being between 5 and 40% by weight relative to the total weight of the composition, and at least one cationic polymer.

2. Composition for washing keratin materials, **characterized in that** it comprises, in a cosmetically acceptable aqueous medium containing at least one anionic detergent surfactant, at least one sorbitan ester of a saturated or unsaturated, linear or branched C₈-C₃₀ fatty acid, oxyethylenated with a number of moles of ethylene oxide of less than or equal to 10, in a sorbitan ester/anionic detergent surfactant weight ratio of between 0.5 and 5, the quantity of anionic detergent surfactant being between 5 and 40% by weight relative to the total weight of the composition, and at least one amphoteric surfactant.

3. Composition according to Claim 1 or 2, **characterized in that** it contains at least one C₈-C₂₀ fatty acid ester of sorbitan oxyethylenated with a number of moles of ethylene oxide of less than or equal to 10.

4. Composition according to Claim 1, 2 or 3, **characterized in that** the oxyethylenated sorbitan ester contains 3 to 8 mol of ethylene oxide.

5. Composition according to any one of Claims 1 to 4, **characterized in that** the sorbitan ester is a sorbitan monoester.

6. Composition according to any one of Claims 1 to 5, **characterized in that** the sorbitan ester is chosen from oxyethylenated sorbitan monolaurate (4EO), oxyethylenated sorbitan monostearate (4EO) and oxyethylenated sorbitan monooleate (5EO).

7. Composition according to any one of Claims 1 to 6, **characterized in that** the sorbitan ester is present in proportions of from 0.5% to 20% and preferably from 2% to 15% relative to the weight of the said composition.

8. Composition according to any one of Claims 1 to 7, **characterized in that** it also comprises a detergent surfactant chosen from nonionic surfactants other than oxyethylenated C₈-C₃₀ fatty acid esters of sorbitan with a number of moles of ethylene oxide of less than or equal to 10, and amphoteric and zwitterionic surfactants.

9. Composition according to any one of Claims 1 to 8, **characterized in that** it also contains one or more cationic surfactants.

10. Composition according to any one of Claims 1 to 9, **characterized in that** it also contains one or more silicones.

11. Composition according to any one of Claims 1 to 10, **characterized in that** it also contains one or more cationic or amphoteric polymers.

12. Composition according to Claim 11, **characterized in that** it contains at least one cationic polymer chosen from cellulose ether derivatives comprising quaternary ammonium groups, polysaccharides, in particular cationic guar gums and cyclopolymers of methyldiallylammonium or of dimethyldiallylammonium.

13. Composition according to any one of Claims 1 to 12, **characterized in that** it also contains viscosity regulators, such as electrolytes, for instance sodium chloride, thickeners, for instance cellulose derivatives, guar gum, hydroxypropyl guar gums, scleroglucans, xanthan gum and amphiphilic polymers comprising at least one fatty chain.

14. Composition according to any one of Claims 1 to 13, **characterized in that** it also contains one or more adjuvants chosen from fragrances, preserving agents, foam synergists, sequestrents, foam stabilizers, propellants, dyes, antidandruff agents, ceramides, vitamins or provitamins, hydroxy acids, acidifying or basifying agents, plant, mineral or animal oils, synthetic oils such as polyisobutenes and polydecenes, fatty acid esters, pseudoceramides and nacreous agents.

15. Use of at least one C₈-C₃₀ fatty acid ester of sorbitan having a number of moles of ethylene oxide of less than or equal to 10, with the aim of reducing the eye-irritant potential and of improving the thickening of compositions for washing keratin materials comprising a detergent surfactant.

16. Use according to Claim 15, in a shampoo.

17. Process for washing keratin materials, **characterized by** the application of at least one composition as defined in any one of Claims 1 to 14 to these materials, followed by rinsing of the treated materials after an optional exposure time.

## Patentansprüche

1. Zusammensetzung zur Reinigung von Keratinsubstanzen, **dadurch gekennzeichnet, dass** sie in einem kosmetisch akzeptablen, wässrigen Medium, das mindestens einen anionischen reinigenden grenzflächenaktiven Stoff enthält, mindestens einen Ester von Sorbitan und einer gesättigten oder ungesättigten, geradkettigen oder verzweigten C₈-₃₀-Fettsäure, der mit einer Molzahl von Ethylenoxid kleiner oder gleich 10 ethoxyliert ist, in einem Gewichtsverhältnis anionischer reinigender grenzflächenaktiver Stoff/Sorbitanester im Bereich von 0,5 bis 5 und mindestens ein kationisches Polymer enthält, wobei der Mengenanteil des anionischen reinigenden grenzflächenaktiven Stoffes im Bereich von 5 bis 40 Gew.-%, bezogen auf das Gesamtgewicht der Zusammensetzung, liegt.

2. Zusammensetzung zur Reinigung von Keratinsubstanzen, **dadurch gekennzeichnet, dass** sie in einem kosmetisch akzeptablen, wässrigen Medium, das mindestens einen anionischen reinigenden grenzflächenaktiven Stoff enthält, mindestens einen Ester von Sorbitan und einer gesättigten oder ungesättigten, geradkettigen oder verzweigten C₈₋₃₀-Fettsäure, der mit einer Molzahl von Ethylenoxid kleiner oder gleich 10 ethoxyliert ist, in einem Gewichtsverhältnis anionischer reinigender grenzflächenaktiver Stoff/Sorbitanester im Bereich von 0,5 bis 5 und mindestens einen amphoteren grenzflächenaktiven Stoff enthält, wobei der Mengenanteil des anionischen reinigenden grenzflächenaktiven Stoffes im Bereich von 5 bis 40 Gew.-%, bezogen auf das Gesamtgewicht der Zusammensetzung, liegt.

3. Zusammensetzung nach Anspruch 1 oder 2, **dadurch gekennzeichnet, dass** sie mindestens einen Sorbitanester einer C₈₋₂₀-Fettsäure enthält, der mit einer Molzahl Ethylenoxid von 10 oder darunter ethoxyliert ist.

4. Zusammensetzung nach Anspruch 1, 2 oder 3, **dadurch gekennzeichnet, dass** der ethoxylierte Sorbitanester 3 bis 8 mol Ethylenoxid enthält.

5. Zusammensetzung nach einem der Ansprüche 1 bis 4, **dadurch gekennzeichnet, dass** der Sorbitanester ein Sorbitanmonoester ist.

6. Zusammensetzung nach einem der Ansprüche 1 bis 5, **dadurch gekennzeichnet, dass** der Sorbitanester unter ethoxyliertem (4 EO) Sorbitanmonolaurat, ethoxyliertem (4 EO) Sorbitanmonostearat und ethoxyliertem (5 EO) Sorbitanmonooleat ausgewählt ist.

7. Zusammensetzung nach einem der Ansprüche 1 bis 6, **dadurch gekennzeichnet, dass** der Sorbitanester in Mengenanteilen von 0,5 bis 20 % und vorzugsweise 2 bis 15 Gew.-% der Zusammensetzung enthalten ist.

8. Zusammensetzung nach einem der Ansprüche 1 bis 7, **dadurch gekennzeichnet, dass** sie ferner einen reinigenden grenzflächenaktiven Stoff enthält, der unter den nichtionischen grenzflächenaktiven Stoffen, die von den Sorbitanestern von C₈₋₃₀-Fettsäuren, die mit einer Ethylenoxidmolzahl von höchstens 10 ethoxyliert sind, verschieden sind, amphoteren grenzflächenaktiven Stoffen oder zwitterionischen grenzflächenaktiven Stoffen ausgewählt sind.

9. Zusammensetzung nach einem der Ansprüche 1 bis 8, **dadurch gekennzeichnet, dass** sie ferner einen oder mehrere kationische grenzflächenaktive Stoffe enthält.

10. Zusammensetzung nach einem der Ansprüche 1 bis 9, **dadurch gekennzeichnet, dass** sie ferner ein oder mehrere Silicone enthält.

11. Zusammensetzung nach einem der Ansprüche 1 bis 10, **dadurch gekennzeichnet, dass** sie ferner ein oder mehrere kationische oder amphotere Polymere enthält.

12. Zusammensetzung nach Anspruch 11, **dadurch gekennzeichnet, dass** sie mindestens ein kationisches Polymer enthält, das unter den Celluloseetherderivaten mit quartären Ammoniumgruppen, Polysacchariden, insbesondere kationischen Guargummen, und Methyldiallylammoniumcyclopolymeren oder Dimethyldiallylammoniumcyclopolymeren ausgewählt ist.

13. Zusammensetzung nach einem der Ansprüche 1 bis 12, **dadurch gekennzeichnet, dass** sie ferner Viskositätsregler, beispielsweise Elektrolyte, wie Natriumchlorid, Verdickungsmittel, wie Cellulosederivate, Guargummi, hydroxypropylierte Guargummen, Skleroglucane, Xanthangummi und amphiphile Polymere mit mindestens einer Fettkette enthält.

14. Zusammensetzung nach einem der Ansprüche 1 bis 13, **dadurch gekennzeichnet, dass** sie ferner einen oder mehrere Zusatzstoffe enthält, die unter den Parfums, Konservierungsmitteln, Stoffen, die hinsichtlich der Schaumbildung synergistisch wirken, Maskierungsmitteln, Schaumstabilisatoren, Treibmitteln, Farbmitteln, Antischuppenmitteln, Ceramiden, Vitaminen, Provitaminen, Hydroxysäuren, Ansäuerungsmitteln, Alkalisierungsmitteln, pflanzlichen Ölen, Mineralölen, tierischen Ölen, synthetischen Ölen, wie Polyisobutenen und Polydecenen, Fettsäureestern, Pseudoceramiden und Perlglanzstoffen ausgewählt sind.

15. Verwendung mindestens eines Sorbitanesters einer C₈₋₃₀-Fettsäure mit einer Ethylenoxidmolzahl von höchstens 10, um die mögliche reizende Wirkung auf die Augen zu vermindern und die Verdickung der Zusammensetzungen zur Reinigung von Keratinsubstanzen, die einen reinigenden grenzflächenaktiven Stoff enthalten, zu verbessern.

16. Verwendung nach Anspruch 15 in einem Haarwaschmittel.

17. Verfahren zur Reinigung von Keratinsubstanzen, **dadurch gekennzeichnet, dass** auf die Keratinsubstanzen mindestens eine Zusammensetzung nach einem der Ansprüche 1 bis 14 aufgetragen und anschließend die behandelten Keratinsubstanzen gegebenenfalls nach einer Einwirkzeit gespült werden.
